# EUROPEAN PATENT APPLICATION

(11) **EP 3 912 658 A1**
(43) Date of publication of application: **24.11.2021**
(21) Application number: 19910633.7
(22) Date of filing: 27.12.2019
(51) Int. Cl.: A61M 5/145, A61M 5/30

(54) **NEEDLELESS SYRINGE**

(30) Priority: 16.01.2019 JP 2019005168
(71) Applicant: Daicel Corporation, Osaka 530-0011 (JP)
(72) Inventor: IGA, Hiromitsu, Tokyo 108-8230 (JP); YAMAMOTO, Yuzo, Tokyo 108-8230 (JP)
(74) Representative: Grünecker Patent- und Rechtsanwälte PartG mbB
(86) International application number: PCT/JP2019/051529
(87) International publication number: WO 2020/149152

(57) **Abstract**

A needleless injector ejects an intended injection substance from an ejection port to a target region in a state in which the ejection port is in contact with a surface of the target region. The needleless injector includes a housing part that has an accommodating space that accommodates the intended injection substance, a nozzle portion that defines a flow path for guiding the intended injection substance accommodated in the housing part to the ejection port, a driving part that imparts ejection energy for ejecting the intended injection substance, a pressurizing portion that pressurizes the intended injection substance accommodated in the accommodating space via a propellant disposed to move or deform in a predetermined direction inside the needleless injector by imparting the ejection energy, and a notification unit that, after the ejection energy is imparted by the driving part and the intended injection substance is delivered into the target region, makes a predetermined notification regarding a first timing for canceling a contact state between the ejection port and the surface of the target region to a user.

## Description

### [Field]

The present disclosure relates to a needleless injector configured to eject an intended injection substance to a target region without using an injection needle.

### [Background]

Although a needleless injector that does not include an injection needle can be exemplified as a device that ejects a liquid chemical and the like to a target region such as an organism, in recent years, the needleless injector has been focused on because of ease of handling, sanitation, and the like and has been developed. In general, there has been implemented a needleless injector having a configuration in which a liquid chemical pressurized by a drive source such as compressed gas and a spring is ejected to a target region and the liquid chemical is ejected to inside of the target region through use of the kinetic energy of the liquid chemical.

The needleless injector disclosed in Patent Document 1 includes a sensor that senses whether a tip of a nozzle is in proper contact with the skin when a liquid chemical and the like are injected into a patient and the like. In this needleless injector, when the sensor senses that the tip of the nozzle is not located at a fixed position suitable for injection of the liquid chemical, control is performed so as not to drive a microj et that jets the liquid chemical. In addition, the needleless injector includes an alarming mechanism, such as a buzzer, that notifies a user that the tip of the nozzle is not located at the fixed position.

### [Citation List]

### [Patent Document]

[Patent Document 1] JP 2006-524120 A

### [Summary]

### [Technical Problem]

In related art techniques, a sensor senses whether a needleless injector and the skin of a patient or the like are in proper contact with each other when a liquid chemical is administered by the needleless injector, and when the needleless injector and the skin of the patient or the like are not in proper contact with each other, a drive source is controlled so as not to eject the liquid chemical.

Here, in order to prevent backflow of a liquid chemical from the skin after injection of the liquid chemical in a needleless injector, it is preferable to maintain a state in which the needleless injector is in contact with the skin for a fixed period of time, rather than to release the needleless injector from the skin immediately after injection of the liquid chemical. This is because since the needleless injector ruptures a portion of the skin with the kinetic energy of the ejected liquid chemical to deliver the liquid chemical to a predetermined site, it is preferable to maintain a state in which the needleless injector and a surface of the skin are in contact with each other during the time required to close the ruptured portion of the skin after jetting of the liquid chemical.

However, the prior art does not mention configurations and the like to ensure time of contact between the needleless injector and the skin after injection of the liquid chemical. As the time of contact between the needleless injector and the skin after injection of the liquid chemical, if a certain period of time is not ensured, there is a problem in that the liquid chemical will flow back to the skin surface.

In view of the problem described above, an object of the present disclosure is to provide a technique that can suppress backflow of an intended injection substance after a needleless injector ejects the intended injection substance.

### [Solution to Problem]

In order to solve the above problem, a needleless injector of the present disclosure adopts a configuration in which, after an intended injection substance is delivered into a target region, a predetermined notification regarding a first timing for canceling a contact state between an ejection port and a surface of the target region is made to a user. With such a configuration, it is possible to ensure a retention time of allowing the needleless injector after delivery of the intended injection substance to contact with the surface of the target region, and therefore, backflow of the intended injection substance can be suppressed after the needleless injector ejects the intended injection substance.

Specifically, the present disclosure is a needleless injector that ejects an intended injection substance from an ejection port into a target region in a state in which the ejection port is in contact with a surface of the target region, and includes a housing part that has an accommodating space that accommodates the intended injection substance, a nozzle portion that defines a flow path for guiding the intended injection substance accommodated in the housing part to the ejection port, a driving part that imparts ejection energy for ejecting the intended injection substance, a pressurizing portion that pressurizes the intended injection substance accommodated in the accommodating space via a propellant disposed to move or deform in a predetermined direction inside the needleless injector by imparting the ejection energy, and a notification unit that, after the ejection energy is imparted by the driving part and the intended injection substance is delivered into the target region, makes a predetermined notification regarding a first timing for canceling a contact state between the ejection port and the surface of the target region to a user.

In the needleless injector, the driving part imparts the ejection energy to the intended injection substance accommodated in the housing part, and thus the intended injection substance is ejected to the target region. In the present application, "ejection" is achieved by imparting the ejection energy to the intended injection substance through the driving part, so that the intended injection substance flows from the housing part to the ejection port.

Further, as the intended injection substance ejected from the needleless injector, predetermined substances including a component expected to have effects in the target region or a component expected to exert a predetermined function in the target region can be exemplified. Thus, as long as at least ejection by the ejection energy described above can be achieved, a physical form of the intended injection substance is not limited. For example, the intended injection substance may be dissolved in liquid, or may be simply mixed without being dissolved in liquid. As one example, the predetermined substance to be sent includes vaccine for intensifying an antibody, a protein for cosmetic enhancement, a cultured cell for hair regeneration, and the like, and is included in a liquid medium in an ejectable manner. The intended injection substance is formed in this way. Note that the medium is preferably a medium that does not hinder the above-mentioned effect and function of the predetermined substance in a state of being injected into the target region. As another method, the medium may be a medium that exerts the above-mentioned effect and function by acting together with the predetermined substance in the state of being injected into the target region.

The intended injection substance ejected needs to rupture the surface of the target region such that the intended injection substance is ejected from the needleless injector to the target region to be delivered into the inside thereof. Thus, at an ejection initial state, the intended injection substance needs to be ejected to the target region at a relatively high speed. In view of this point, as an example, the driving part preferably imparts the ejection energy using a combustion product discharged by combustion of an ignition charge. Note that, as the ignition charge, there may be employed any one of an explosive containing zirconium and potassium perchlorate, an explosive containing titanium hydride and potassium perchlorate, an explosive containing titanium and potassium perchlorate, an explosive containing aluminum and potassium perchlorate, an explosive containing aluminum and bismuth oxide, an explosive containing aluminum and molybdenum oxide, an explosive containing aluminum and copper oxide, an explosive containing aluminum and iron oxide, or an explosive composed of a plurality of these explosives in combination. As characteristics of the above-mentioned ignition charge, the combustion product is gas at a high temperature but does not include a gas component at a room temperature, hence the combustion product is condensed immediately after the ignition. As a result, the driving part can impart the ejection energy in an extremely short period of time. In addition, the driving part may utilize electrical energy of a piezoelectric element or the like or mechanical energy of a spring or the like as the ejection energy instead of the ejection energy caused by the combustion of the ignition charge, and may generate the ejection energy by appropriately combining these forms of energy. For the propellant, a piston disposed to move in a predetermined direction inside the needleless injector, a thin film expanding in a predetermined direction, a corrugation extending in a predetermined direction, and the like can be used.

Here, after the intended injection substance imparted with the ejection energy by the driving part is delivered into the target region, the notification unit makes a predetermined notification regarding the first timing for canceling the contact state between the ejection port and the surface of the target region to a user. The needleless injector can maintain the contact state between the ejection port and the surface of the target region for the retention time until the predetermined notification regarding the first timing is made to the user and therefore can suppress backflow of the intended injection substance.

The notification unit of the needleless injector may continuously generate a signal related to the predetermined notification during a time from the start of the imparting of the ejection energy by the driving part to the first timing. The notification unit makes the predetermined notification to a time when the ejection port may be separated from the surface of the target region, and terminates the predetermined notification after the retention time has elapsed. With this configuration, the needleless injector of the present application can maintain the contact state between the ejection port and the surface of the target region for the retention time until the predetermined notification regarding the first timing made to the user is terminated and therefore can suppress backflow of the intended injection substance.

The needleless injector may further include a storage unit that stores first information pertaining to the target region and the user, and the notification unit may make the predetermined notification based on the first information stored in the storage unit. The first information may include a site of the target region, age and gender of a subject person, and the like. The first information is stored in advance in the storage unit prior to use of the needleless injector. The needleless injector provided with this configuration can determine the retention time based on the first information to make the predetermined notification, and can grasp the first timing at which the user cancels the contact state between the ejection port and the surface of the target region, so that backflow of the intended injection substance can be suppressed.

Here, the needleless injector described above may further include a predetermined sensor that acquires second information pertaining to the target region near the ejection port in the contact state between the ejection port and the surface of the target region, and the notification unit may make the predetermined notification based on the second information acquired by the predetermined sensor. The second information may be moisture, elasticity, thickness of the skin, and the like at the site of the target region. The needleless injector can determine the retention time based on the second information to make the predetermined notification, and can grasp the first timing at which the user cancels the contact state between the ejection port and the surface of the target region, so that backflow of the intended injection substance can be suppressed.

The needleless injector described above may further include a pressure sensor capable of detecting the contact state between the ejection port and the surface of the target region, and the notification unit may make an additional notification to the user so that a pressing force of the ejection port against the surface of the target region is increased when a detected value from the pressure sensor falls below a predetermined first pressure value until the first timing has elapsed from the start of the imparting of the ejection energy by the driving part. The needleless injector provided with this configuration can maintain the contact state between the ejection port and the surface of the target region at a predetermined first pressure value or greater until the retention time has elapsed, so that backflow of the intended injection substance can be suppressed. Here, the predetermined first pressure value is the pressure required to deliver the intended injection substance to the target region during the retention time or to prevent backflow of the intended injection substance delivered.

The driving part of the needleless injector described above is allowed to operate when the detected value from the pressure sensor is a predetermined second pressure value or greater. The predetermined second pressure value is the pressure required to deliver the intended injection substance to the target region without leakage, and, for example, the pressure required to prevent formation of a gap between the target region and the ejection port when a portion of the skin in the target region is ruptured with the kinetic energy of the intended injection substance. Since the needleless injector provided with this configuration can eject the intended injection substance in a state in which the ejection port and the surface of the target region are in contact with each other at the predetermined second pressure value, the intended injection substance can be delivered under the skin in the target region, so that backflow of the intended injection substance can be suppressed.

The needleless injector described above may further include a retreat portion that retreats the nozzle portion to the inside of the needleless injector to cancel the contact state between the ejection port and the surface of the target region when the notification unit makes the predetermined notification.

### [Advantageous Effects of Invention]

According to the technique of the present disclosure, backflow of the intended injection substance can be suppressed after the needleless injector has ejected the intended injection substance.

### [Brief Description of Drawings]

[FIG. 1] FIG. 1 is a diagram illustrating a schematic configuration of a needleless injector.
[FIG. 2] FIG. 2 is a first cross-sectional view of a needleless injector.
[FIG. 3] FIG. 3 is a second cross-sectional view of the needleless injector.
[FIG. 4] FIG. 4 is a diagram illustrating a configuration of a housing of the needleless injector.
[FIG. 5] FIG. 5 is a diagram illustrating a schematic configuration of an injector assembly incorporated in the needleless injector.
[FIG. 6] FIG. 6 is a diagram illustrating a schematic configuration of an actuator incorporated in the needleless injector.
[FIG. 7] FIG. 7 is a diagram illustrating a schematic configuration of a piston incorporated in the needleless injector.
[FIG. 8] FIG. 8 is a diagram illustrating a schematic configuration of an attachment incorporated in the needleless injector.
[FIG. 9] FIG. 9 is a diagram illustrating a schematic configuration of a plunger rod and plunger incorporated in the needleless injector.
[FIG. 10] FIG. 10 is a diagram illustrating a schematic configuration of a container incorporated in the needleless injector.
[FIG. 11] FIG. 11 is a block diagram of a controller of a needleless injector according to a first embodiment.
[FIG. 12] FIG. 12 is a flowchart relating to processing performed by the controller of the needleless injector according to the first embodiment.
[FIG. 13] FIG. 13 is a flowchart relating to processing performed by a controller of a needleless injector according to a second embodiment.
[FIG. 14] FIG. 14 is a block diagram of a controller of a needleless injector according to a third embodiment.
[FIG. 15] FIG. 15 is a diagram illustrating a schematic configuration of a container incorporated in a needleless injector according to a fourth embodiment.
[FIG. 16] FIG. 16 is a block diagram of a controller of the needleless injector according to the fourth embodiment.
[FIG. 17] FIG. 17 is a flowchart relating to processing performed by the controller of the needleless injector according to the fourth embodiment.
[FIG. 18] FIG. 18 is a flowchart relating to the processing performed by the controller of the needleless injector according to the fourth embodiment.
[FIG. 19] FIG. 19 is an enlarged view of the vicinity of a nozzle portion of a container incorporated in a needleless injector according to a fifth embodiment.
[FIG. 20] FIG. 20 is a block diagram of a controller of the needleless injector according to the fifth embodiment.

With reference to the drawings, a needleless injector 1 according to an embodiment of the present disclosure (herein, simply referred to as "injector") is described below. The injector 1 is a needleless injector that implements injection by ejecting an ejection solution, which corresponds to an intended injection substance in the present application, to a target region through use of a combustion energy of an explosive, that is, a device that injects the ejection solution to the target region without using an injection needle.

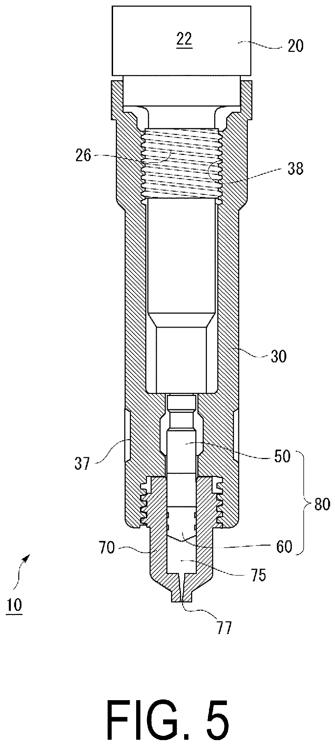

Each of the configurations, combinations thereof, and the like in each embodiment are an example, and various additions, omissions, substitutions, and other changes may be made as appropriate without departing from the spirit of the present invention. The present disclosure is not limited by the embodiments and is limited only by the claims. Note that, in the present embodiment, as terms indicating a relative positional relationship in a longitudinal direction of the injector 1, "distal end side" and "base end side" are used. The "distal end side" indicates a side closer to the distal end of the injector 1 described later, that is, a position closer to an ejection port 77, and the "base end side" indicates a side in an opposite direction to the "distal end side" in a longitudinal direction of the injector 1, that is, a direction to an igniter 22 side of an injector assembly 10 (see FIG. 5 described later).

### <Configuration of injector 1>

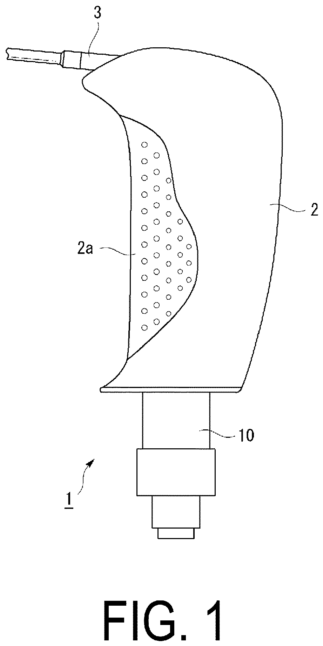

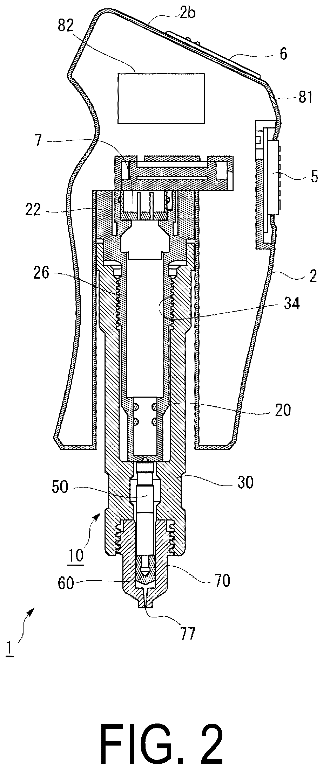

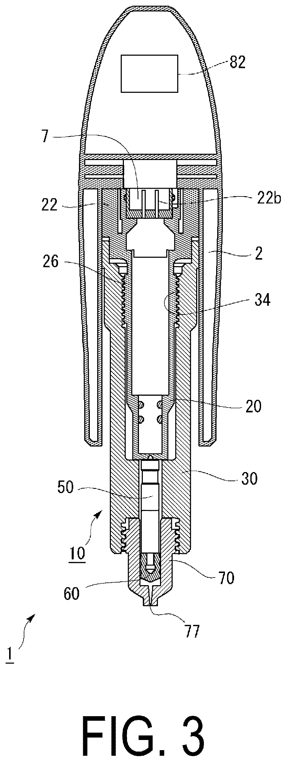

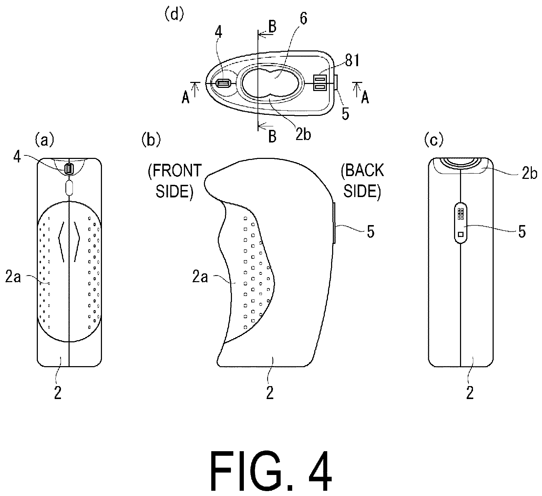

Here, FIG. 1 is a diagram schematically illustrating the appearance of the injector 1. FIG. 2 is a first cross-sectional view of the injector 1, which is an AA cross section in FIG. 4, described below. FIG. 3 is a second cross-sectional view of injector 1, a BB cross section in FIG. 4 described below. The BB cross section is orthogonal to the AA cross section. Note that FIG. 4 is a diagram illustrating a configuration of a housing 2 that is a part of the injector 1. Here, the injector 1 is formed with the injector assembly 10 attached to the housing 2. A power cable 3 for supplying drive current to the igniter 22 in the injector assembly 10 is connected to the housing 2.

Note that, in the following description in the present application, the ejection solution ejected to the target region by the injector 1 is formed of a liquid medium including a predetermined substance, which exerts an effect or a function expected in the target region. In the ejection solution, the predetermined substance may be in a state of being dissolved in liquid being a medium, or may be in a state of being simply mixed instead of being dissolved.

For example, examples of the predetermined substance included in the ejection solution include an organism-derived substance and a substance with a desired bioactivity, which can be ejected to the target region being an organism. For example, examples of the organism-derived substance include DNA, RNA, a nucleic acid, an antibody, and a cell. Examples of the substance with a desired bioactivity include various substances exerting pharmacological or therapeutic effects, which are exemplified by medicines composed of low molecular compounds, proteins, peptides, or the like, a vaccine, an inorganic substance such as metal particles for thermotherapy or radiotherapy, and a carrying body functioning as a carrier. Further, the liquid being the medium of the ejection solution is only required to be a substance suitable for administering the predetermined substance exemplified by those substances to the target region, and may be aqueous or oleaginous, which is not limited. Further, viscosity of the liquid being the medium is not particularly limited as long as the predetermined substance can be ejected by the injector 1.

In the injector 1, the injector assembly 10 is configured to be attachable to and detachable from the housing 2 freely. An accommodating space 75 (see FIG. 5) formed between a container 70 and a plunger 80 in the injector assembly 10 is filled with ejection solution during a preparation stage before the operation of the injector 1. The injector assembly 10 is a unit that is replaced each time the ejection solution is ejected. The injector assembly 10 will be described in detail below.

On the other hand, the housing 2 has a grip portion 2a formed to be gripped by a user of the injector 1 in use, and is provided with a plurality of switches for operating the injector 1 to eject the ejection solution. Note that the injector 1 is configured to be capable of being held and operated by one hand of the user. In this context, the housing 2 will be described with reference to FIG. 4. In FIG. 4, (a) illustrates the outer appearance of the housing 2 as viewed from the front side, (b) illustrates the outer appearance of the housing 2 as viewed from one side, (c) illustrates the outer appearance of the housing 2 as viewed from the back side, and (d) illustrates the outer appearance of the housing 2 as viewed from the upper side. Here, "front side" indicates a portion positioned on the distal side of the user holding the housing 2, which is the left side in FIG. 4(b), and "back side" indicates a portion positioned on the proximal side of the user holding the housing 2, which is the right side in FIG. 4(b). Thus, when the user holds the housing 2 with one hand, fingertips rest on the front side of the distal housing 2 which is the distal side, and the wrist is in the vicinity of the back side of the housing 2 which is the proximal side. The "upper side" is a portion of the injector 1 on the base end side.

Considering such a way of holding by the user, the grip portion 2a is provided at a front side portion of the housing 2 so that the user can easily rest his or her fingertips thereon. The grip portion 2a is provided with a plurality of dimples making the user's fingertips even easier to be rested thereon. Furthermore, the grip portion 2a has gentle recesses and protrusions on the front side of its outer shell (see (b) in FIG. 4) so that the user's forefinger and middle finger can be easily rested thereon, for the sake of more stable holding of the housing by the user.

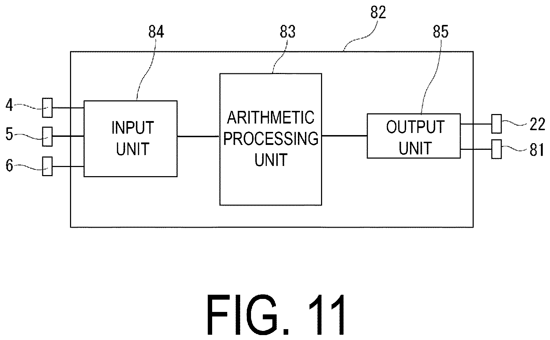

Further, the housing 2 is provided with a first switch 5 and a second switch 6 that are two operating switches for operating the injector 1. The first switch 5 and the second switch 6 are connected to a controller 82 (see FIG. 11) such as a microcomputer disposed in the housing 2, and the controller 82 controls the supply of ignition current to the igniter 22 based on a signal from each switch, thereby controlling an operation of the injector 1. The first switch 5 is a sliding switch provided on the back side of the housing 2, the sliding direction of which being an upward and downward direction of the housing 2 (direction between the distal end and the base end). The first switch 5 is constantly biased in the upward direction. The user can achieve a standby state of the injector 1 by continuously sliding the first switch 5 downward (toward the distal end side) for a predetermined period of time against the biasing force. The standby state is a state in which the injector 1 is ready to eject the ejection solution. When a user makes an additional operation in this state, the ejection is implemented.

The second switch 6 is a press type switch provided on an inclined surface 2b on the upper side of the housing 2. The user can press the second switch 6 in a direction toward the inner side of the housing 2. The controller 82 is configured to supply an ignition current to the igniter 22 in response to the pressing operation on the second switch 6 while the injector 1 is in the standby state as a result of the operation on the first switch 5 described above. A connector 4 to which the power cable 3 is connected is provided on the front side of the inclined surface 2b on the upper side of the housing 2. In the present embodiment, the connector 4 is a USB connector, and the power cable 3 is freely attachable to and detachable the housing 2.

Note that, as described above, in the present embodiment, the power for actuating the igniter 22 is supplied from the outside through the power cable 3. Alternatively, a battery for supplying such power may be provided inside the housing 2. In this case, the housing 2 can be repeatedly used while replacing the injector assembly 10, until the battery runs out. When the battery runs out, the battery may be replaced.

A schematic configuration of the injector assembly 10 is illustrated in FIG. 5. The injector assembly 10 is attached to the housing 2 to form the injector 1, as illustrated in FIGS. 2 and 3. Specifically, the injector assembly 10 is an assembly including an actuator 20, an attachment 30, the container 70, and the plunger 80. How the injector assembly 10 is assembled will be described below.

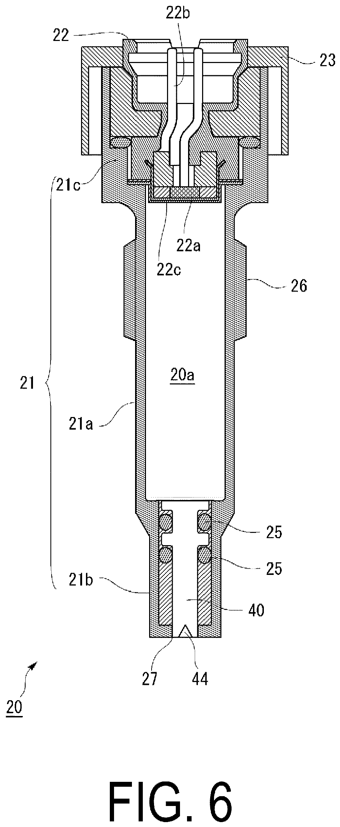

First of all, the actuator 20 will be described with reference to FIG. 6. The actuator 20 has a body 21 formed in a cylindrical shape. The body 21 includes a center portion 21a in the center thereof, a distal end portion 21b on the distal end side thereof, and a base end portion 21c on the base end side thereof. The distal end portion 21b, the center portion 21a, and the base end portion 21c of the body 21 have their internal spaces in communication with each other. The distal end portion 21b has an opening 27 on the distal end side. The igniter 22, which is an electric igniter that generates energy for ejection through combustion of an ignition charge 22a, is attached to the base end portion 21c of the body 21 via a cap 23. The igniter 22 has an ignition pin 22b to which ignition current is supplied from the outside. The ignition pin 22b is coupled to a socket 7 on the side of the housing 2 in a state in which the injector assembly 10 is attached to the housing 2. The attachment state of the igniter 22 to the body 21 is determined such that a combustion product generated by the operation of the igniter 22 is discharged toward the center portion 21a of the body 21. Specifically, the igniter 22 is attached to the base end portion 21c of the body 21 to have a discharge surface 22c, from which the combustion product is discharged, directed toward the center portion 21a.

Herein, a combustion energy used in the igniter 22 for the ignition charge is an energy for the injector 1 to eject the ejection solution to the target region. Note that, examples of the ignition charge include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), or an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. An explosive other than these may be used as the ignition charge as long as appropriate ejection of the ejection solution can be performed.

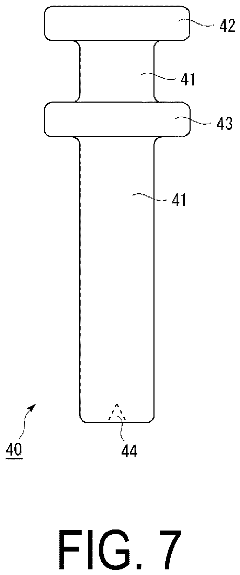

The internal space of the center portion 21a of the body 21 serves as a combustion chamber 20a into which a combustion product is discharged from the igniter 22. Furthermore, a male thread portion 26 is formed in a part of the outer surface of the center portion 21a. The male thread portion 26 is configured to mate with a female thread portion 38 of the attachment 30 described below. The effective lengths of the male thread portion 26 and the female thread portion 38 are determined to guarantee sufficient coupling force therebetween. The internal space of the distal end portion 21b adjacent to the center portion 21a is formed in a cylindrical shape in which a piston 40 is slidably provided and O rings 25 serving as a sealing member are also provided. The piston 40 is made of metal, has a shaft member 41, is provided with a first flange 42 on the base end side thereof, and is further provided with a second flange 43 in the vicinity of the first flange 42, as illustrated in FIG. 7. The first flange 42 and the second flange 43 have a disc shape, and have the same diameter. The O rings 25 include one disposed between the first flange 42 and the second flange 43 and one disposed on another side of the second flange 43. A recess portion 44 having a predetermined size is formed in a distal end surface of the shaft member 41. In a state where the piston 40 is disposed in the internal space of the distal end portion 21b before the actuation of the actuator 20, the first flange 42, which serves as a surface receiving pressure of the combustion product from the igniter 22, is exposed on side of the combustion chamber 20a, and the distal end of the shaft member 41 of the piston 40 is inserted into the opening 27.

Then, when the igniter 22 is activated and the combustion product is discharged into the combustion chamber 20a and thus the pressure therein rises, the first flange 42 receives the pressure, resulting in the piston 40 sliding toward the distal end side. Thus, the actuator 20 has a mechanism with the igniter 22 serving as an actuation source and the piston 40 serving as an output unit. Since the second flange 43 has a larger diameter than the opening 27, the distance by which the piston 40 can slide is limited. Thus, the distance by which the shaft member 41 of the piston 40 can protrude from the distal end surface of the distal end portion 21b of the body 21 is limited. Further, the piston 40 may be formed of a resin, and in such case, metal may be used together for a part to which heat resistance and pressure resistance are required.

Additionally, as an alternative mechanism to adjust the pressure applied to the piston 40, the combustion chamber 20a of the actuator 20 may be further provided with a gas generating agent that is burned by the combustion product from the igniter 22 to produce gas. The agent may be disposed, for example, at a location that may be exposed to the combustion product from the igniter 22. Further, as another method, the gas generating agent may be disposed in the igniter 22 as disclosed in WO 01/031282, JP 2003-25950 A, and the like. As one example of the gas generating agent, there may be exemplified a single base smokeless explosive formed of 98 mass% of nitrocellulose, 0.8 mass% of diphenylamine, and 1.2 mass% of potassium sulfate. Further, various types of gas generating agents used in a gas generator for an air bag and a gas generator for a seat belt pretensioner may be used. A combustion completion time period of the gas generating agent can be changed by adjusting a dimension, a size, a shape, and particularly, a surface shape of the gas generating agent at the time of being disposed in the combustion chamber 20a or the like. With this, the pressure applied to the piston 40 can be adjusted to a desired pressure.

The piston 40 is an example of a propellant disposed to move in a predetermined direction inside the injector 1. The injector 1 may include another propellant instead of the piston 40. For example, like a thin film inflated in a predetermined direction by combustion gas as disclosed in US 2006/0,089,595 A, or a corrugation extending in a predetermined direction by combustion gas as disclosed in US 7,063,019, a propellant disposed to deform in a predetermined direction inside the injector 1 can also be used.

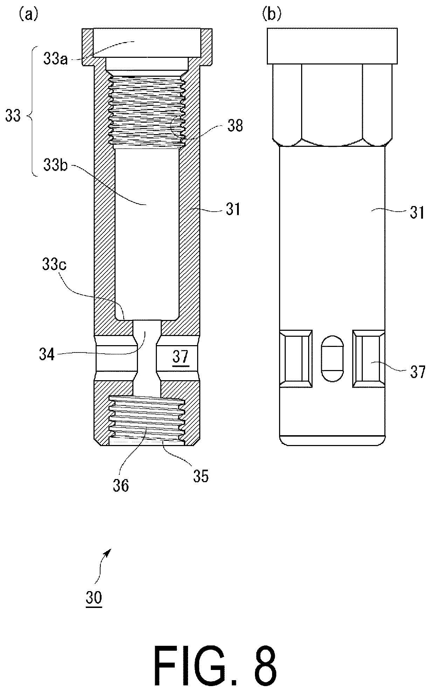

Next, the attachment 30 will be described based on FIG. 8. Note that FIG. 8 includes the diagram (a) on the left side that is a cross-sectional view of the attachment 30, and the diagram (b) on the right side that is an external view of the attachment 30. The attachment 30 is a member for attaching the actuator 20, the plunger 80, and the container 70 as illustrated in FIG. 5. For the body 31 of the attachment 30, for example, it is possible to use known nylon 6-12, polyarylate, polybutylene terephthalate, polyphenylene sulfide or liquid crystal polymer, polycarbonate, a mixture of polycarbonate and an acrylonitrile-butadiene-styrene copolymer (ABS resin), and the like. Further, a filler such as glass fibers and glass filler may be contained in those resins. 20 to 80 mass% of glass fibers may be contained in polybutylene terephthalate, 20 to 80 mass% of glass fibers may be contained in polyphenylene sulphide, or 20 to 80 mass% of minerals may be contained in a liquid crystal polymer.

The internal space of the body 31 includes a first region 33, extending from the base end side to the center, where the actuator 20 is disposed as illustrated in FIG. 5. The first region 33 includes a region 33a on the base end side where the base end portion 21c of the actuator 20 is generally positioned, and a region 33b on the distal end side of the first region 33 where the center portion 21a and the distal end portion 21b of the actuator 20 are generally positioned. The region 33b has a smaller diameter than the region 33a. The female thread portion 38 is disposed on the inner wall surface at a portion of the region 33b close to the region 33a. The female thread portion 38 is formed to engage with the male thread portion 26 provided on the center portion 21a of the actuator 20.

The internal space of the body 31 further includes a second region 34 in communication with the first region 33. The second region 34 is a region in which the plunger 80 is generally disposed as illustrated in FIG. 5, and is a hollow region formed in a cylindrical shape extending along the axial direction of the body 31. The second region 34 has one end in communication with the region 33b of the first region 33. The second region 34 has a diameter smaller that is smaller than the diameter of the region 33b, and enables a sliding movement of the plunger 80. A through hole 37 extends from a side outer surface of the attachment 30 to the second region 34, to be formed through the body 31. Through the through hole 37, the user can check the status (such as whether the injector assembly 10 is before or after being actuated, for example) of the plunger 80 in the injector assembly 10 from the outside (see FIG. 1).

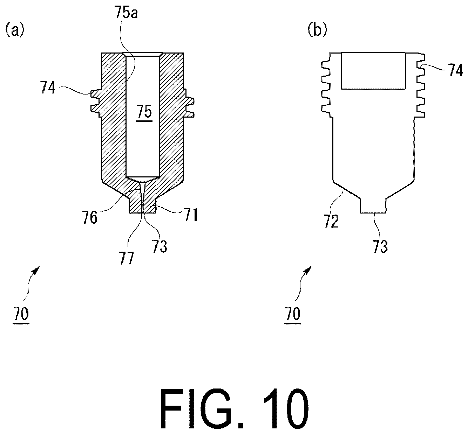

The internal space of the body 31 further includes a third region 35 in communication with the second region 34. The third region 35 is a region in which a part of the container 70 is generally disposed as illustrated in FIG. 5, and has one end in communication with the second region 34, and has the other end open to the distal end surface of the attachment 30. A female thread portion 36 for attachment to the container 70 is formed in the third region 35. The female thread portion 36 is screwed with a male thread portion 74 of the container 70 illustrated in FIG. 10 described below, whereby the attachment 30 and the container 70 are coupled to each other.

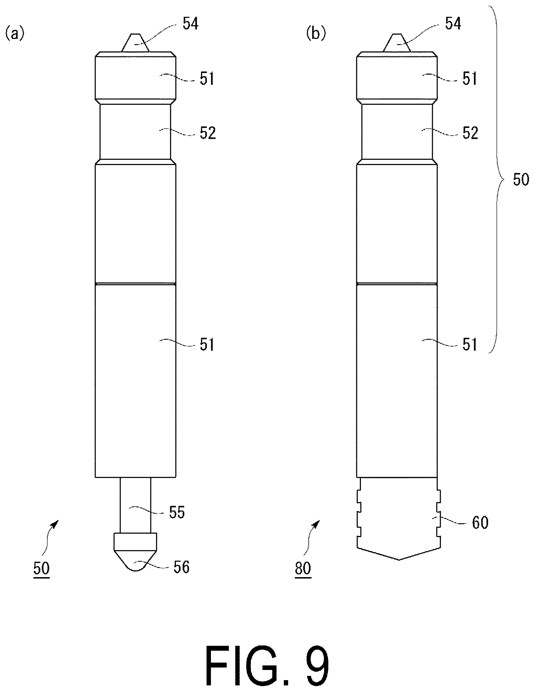

Next, the plunger 80 will be described based on FIG. 9. FIG. 9 includes the diagram (a) on the left side that is an external view of a plunger rod 50, which is one of the components of the plunger 80, and a diagram (b) on the right side that is an external view of the plunger 80. The plunger 80 is a member that pressurizes the ejection solution by energy received from the piston 40, and a resin material suitable for the pressurization (for example, a resin material similar to that used for the attachment 30) can be used for the plunger rod 50. The plunger rod 50 includes a shaft member 51, and has a base end side end surface provided with a protrusion 54. The protrusion 54 is shaped and sized to be capable of fitting in the recess portion 44 of the shaft member of the piston 40 of the actuator 20, when the plunger 80 is incorporated in the injector assembly 10. A diameter reduced portion 52 which narrows to less than the diameter of another shaft member 51 is provided partway through the shaft member 51 and near the base end.

Further, in the plunger rod 50, a protrusion 56 is provided to a distal end side of the shaft member 51 with a neck portion 55 with a smaller diameter than the shaft member 51 provided in between. The protrusion 56 is shaped like a weight to have a diameter being greater than the diameter of the neck portion 55 near a portion to be connected with the neck portion 55 and reducing toward the distal end side. The maximum diameter of the protrusion 56 is smaller than the diameter of the shaft member 51. A stopper portion 60 formed of an elastic member such as rubber is attached to the neck portion 55 and the protrusion 56, whereby the plunger 80 is formed (see FIG. 9(b)). An attachment hole (not illustrated) is formed in the stopper portion 60, and engages with the neck portion 55 and the protrusion 56, so that the stopper portion 60 is less likely to be detached from the plunger rod 50.

Specific examples of materials of the stopper portion 60 include butyl rubber and silicon rubber. Further, there may be exemplified a styrene-based elastomer or a hydrogenated styrene-based elastomer, or a substance obtained by mixing a styrene-based elastomer or a hydrogenated styrene-based elastomer with polyolefin such as polyethylene, polypropylene, polybutene, and an α-olefin copolymer, oil such as liquid paraffin and process oil, or a powder inorganic substance such as talc, cast, and mica. Further, as the material of the stopper portion 60, a polyvinyl chloride-based elastomer, an olefin-based elastomer, a polyester-based elastomer, a polyamide-based elastomer, a polyurethane-based elastomer, various rubber materials (particularly, a vulcanized material) such as natural rubber, isoprene rubber, chloroprene rubber, nitrile butadiene rubber, and styrene butadiene rubber, or a mixture thereof may be employed. Furthermore, the stopper portion 60 pressurizes the ejection solution by sliding within the container 70 described below. Thus, a surface of the stopper portion 60 and an inner wall surface 75a of the accommodating space 75 of the container 70 may be coated or processed using various matters, to guarantee/adjust slidability between the stopper portion 60 and the inner wall surface 75a of the accommodating space 75 of the container 70. Examples of the coating agent may include polytetrafluoroethylene (PTFE), silicon oil, diamond-like carbon, nano diamond, and the like.

Next, the container 70 will be described based on FIG. 10. Note that FIG. 10 includes the diagram (a) on the left side that is a cross-sectional view of the container 70, and the diagram (b) on the right side that is an external view of the container 70. The container 70 is a member containing an ejection solution to be pressurized by the plunger 80, and is a member that defines a flow path for injecting the pressurized ejection solution to the target region. In view of this, a resin material (a resin material of the same type as the attachment 30 for example) may be used for forming the container 70.

The container 70 includes an accommodating space 75, in which the stopper portion 60 of the plunger 80 are movable, accommodating the ejection solution, and a nozzle portion 71 including a flow path 76 connecting the accommodating space 75 to the outside of the container 70. The nozzle portion 71 has a columnar outer circumference on the distal end side. Note that in the injector assembly 10, as illustrated in FIG. 5, a positional relationship between the plunger 80 and the container 70 is determined so that the stopper portion 60 of the plunger 80 can slide within the accommodating space 75 in a direction toward the nozzle portion 71 (direction toward the distal end side). The ejection solution is sealed in a space defined by stopper portion 60 of the plunger 80 and the container 70. The flow path of the container 70 opens in a distal end surface 73 of the nozzle portion 71, so that the ejection port 77 is formed. Thus, when the plunger 80 slides within the accommodating space 75, the ejection solution accommodated in the accommodating space 75 is pressurized to be ejected from the ejection port 77 through the flow path 76.

The flow path 76 provided in the container 70 has a diameter smaller than the inner diameter of the accommodating space 75. With this configuration, the ejection solution that has been applied with a high pressure is ejected to the outside through the ejection port 77. The male thread portion 74 for attaching the container 70 to the attachment 30 is formed on the base end side of the container 70. The male thread portion 74 is screwed with the female thread portion 36 of the attachment 30.

Note that the profile on the distal end side of the stopper portion 60 of the plunger 80 is shaped to substantially match the profile of the inner wall surface 75a near a portion where the accommodating space 75 and the flow path 76 are connected to each other (the deepest part of the accommodating space 75). With this configuration, a smallest possible gap can be formed between the stopper portion 60 and the inner wall surface 75a of the container 70 when the plunger 80 slides for ejecting the ejection solution and reaches the deepest part of the accommodating space 75, whereby the ejection solution can be prevented from wastefully remaining in the accommodating space 75. However, the shape of the stopper portion 60 is not limited to a particular shape as long as desired effects can be obtained with the injector 1 according to the present embodiment.

Now, how the injector assembly 10 is assembled will be described. In a state where the stopper portion 60 of the plunger 80 is inserted to the deepest part of the accommodating space 75 of the container 70, the plunger 80 is retracted with the ejection port 77 of the container 70 in communication with the ejection solution. The stopper portion 60 and the inner wall surface 75a of the accommodating space 75 are suitably in close contact with each other, the retraction action will produce negative pressure in the accommodating space. Thus, the accommodating space 75 can be filled with the ejection solution through the ejection port 77. In this process, the plunger 80 is retracted to an extent enough for making the part of the plunger 80 (plunger rod 50) protruding from the container 70 pass through the second region 34 to reach the first region 33 (the region 33b illustrated in FIG. 8), when the container 70 is attached to the attachment 30 in this state.

After the container 70 filled with ejection solution in the accommodating space 75 is attached to the attachment 30, the actuator 20 is inserted to the attachment 30 from the side of the first region 33. The actuator 20 is inserted until the distal end surface of its distal end portion 21b comes into contact with a distal end surface 33c of the region 33b of the attachment 30 (see FIG. 8). Then, in this process, the male thread portion 26 provided to the center portion 21a of the actuator 20 is screwed with the female thread portion 38 of the attachment 30, whereby the actuator 20 and the attachment 30 are suitably coupled to each other. Furthermore, in this process, the recess portion 44 of the shaft member 41 of the piston 40, which is incorporated in the actuator 20, engages with the protrusion 54 of the shaft member 51 of the plunger 80, and the plunger 80 is pushed by the piston 40 toward the distal end side. Note that, a fixing force of the piston 40 in the distal end portion 21b of the actuator 20 is set to an extent that the piston 40 can slide in the distal end portion 21b in a sufficiently smooth manner by a pressure received from the combustion product produced by the igniter 22, and to an extent that the piston 40 can suitably resist force received from the plunger 80 so that the position of the piston 40 is not displaced when the injector assembly 10 is assembled. Alternatively, a stopper may be formed at an intended position of the piston 40, so that the top surface of the first flange 42 of the piston 40 faces the combustion chamber 20a of the actuator 20 and is not displaced toward the combustion chamber 20a as illustrated in FIG. 6. In order to prevent movement of the piston 40 when the actuator 20 and the plunger 80 are assembled to the attachment 30, an opening diameter of the opening 27 of the actuator 20 is made smaller than a diameter of a first flange 40 of the plunger 80, so that the plunger 80 does not push the piston 40 toward the base end portion, and the plunger 80 may be positioned by hitting the first flange 40 against a distal end side end surface of the actuator 20.

Thus, when the actuator 20 is attached to the attachment 30 to which the container 70 and plunger 80 are attached as described above, the plunger 80 is pushed to move from the piston 40 toward the distal end side, whereby the plunger 80 is positioned at a predetermined position within the container 70. Note that, in response to pressing of the plunger 80, a part of the ejection solution is discharged from the ejection port 77.

When the plunger 80 is thus positioned at the final position as described above, formation of the injector assembly 10 is completed. In this injector assembly 10, the position of the stopper portion 60 of the plunger 80 in the accommodating space 75 of the container 70 is mechanically determined. The final position of the stopper portion 60 is a position uniquely determined in the injector assembly 10, and hence an amount of the ejection solution that is finally stored in the accommodating space 75 in the injector assembly 10 can be a predetermined amount determined in advance.

The injector assembly 10 thus configured can be loaded into the housing 2 with the ignition pin 22b of the igniter 22 fitted into the socket 7 on the housing 2, whereby the injector 1 is prepared to be usable (see FIGS. 1 to 3). The user holds the housing 2 of such injector 1 with one hand and slides the first switch 5 located on the back side of the housing 2 for a predetermined period of time, putting the injector 1 in the standby state. In this state, when the user presses the second switch 6 with the ejection port 77 being in contact with the target region, the igniter 22 is actuated, and the ejection solution is pressurized via the piston 40 and the plunger 80. Thus, the ejection is implemented, and the ejection solution is injected into the target region.

### <Predetermined notification by injector>

### <First embodiment>

Here, a first embodiment of the injector 1 will be described with reference to FIG. 11. FIG. 11 is a block diagram of the controller 82 included in the injector 1 according to the present embodiment. As illustrated in FIGS. 2 and 3, the controller 82 is disposed inside the housing 2 and performs various controls. The controller 82 has an arithmetic processing unit 83 configured of a CPU and the like, an input unit 84 through which electric power, data, and an input signal from a switch are input, and an output unit 85 that outputs a control signal and the like. The connector 4 is connected to the input unit 84, and power is input through the power cable 3. In addition, the first switch 5 and the second switch 6 are connected to the input unit 84, and the input signals from these switches are input to the input unit 84. The igniter 22 and a speaker 81 are connected to the output unit 85. As illustrated in FIGS. 2 and 4(d), the speaker 81 is disposed between the first switch 5 and the second switch 6. The controller 82 operates by the power supplied from the connector 4, and controls the igniter 22 and the speaker 81 based on the input signals from the first switch 5 and the second switch 6. Furthermore, the controller 82 controls the speaker 81 to make various notifications (predetermined notifications or additional notifications) to the user by output of effect sounds such as buzzer, voices, and the like. Instead of the speaker 81, a lamp, a display device, or the like that displays characters, numbers, and the like may be provided in the housing 2 of the injector 1, and the controller 82 may control the lamp, the display device, or the like to make various notifications on the user. When the injector 1 includes a lamp, the controller 82 controls the lamp to illuminate or flash the lamp for a predetermined notification. When the injector 1 includes a display device, the controller 82 controls the display device to display a character such as "end" for the predetermined notification. A combination of two or more of a speaker, a lamp, and a display device is provided in the housing 2, and the controller 82 may control these devices and make various notifications to the user.

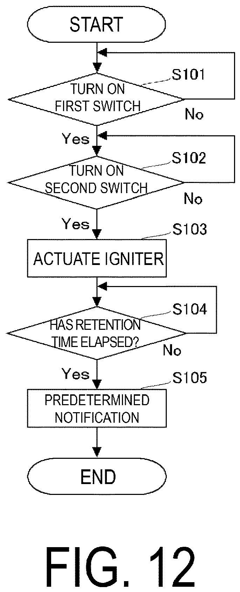

The controller 82 functions as a notification unit that makes the predetermined notification regarding a first timing for canceling the contact state between the ejection port 77 and the surface of the target region to the user. The predetermined notification will be described below. FIG. 12 is a flowchart illustrating processing performed by the controller 82 during the operation of the injector 1. First, the controller 82 determines, based on the input signal, whether the first switch 5 has been turned on (step S101). The first switch 5 is turned on so that the injector 1 is in the standby state. When the controller 82 determines that the first switch is on (Yes in step S101), the controller 82 determines whether the second switch 6 has been turned on based on the input signal (step S102). When the controller 82 detects that the second switch 6 has been turned on (Yes in step S102), the controller 82 supplies ignition current to the igniter 22 and actuates the igniter 22 (step S103).

When the igniter 22 is actuated, the piston 40 slides toward the distal end side to push the plunger 80 toward the distal end side, and ejection energy is imparted to the ejection solution. This causes the ejection solution to be ejected from the ejection port 77. A portion of the skin in the target region is ruptured with the kinetic energy of the ejection solution, and the ejection solution is delivered under the skin. The injector 1 can suppress backflow of the ejection solution by retaining a state in which the ejection port 77 and the skin are in contact with each other during the time required to close a rupture portion of the skin after ejection of the ejection solution. A retention time of retaining the state in which the ejection port 77 and the skin are in contact with each other after ejection is a time required to close the rupture portion of the skin after ejection of the ejection solution and is a time required to suppress backflow of the ejection solution. The retention time is preset based on data such as, for example, a site of the target region and age and gender of a subject person for the ejection solution. The retention time may be set based on the moisture, elasticity, and skin thickness of the target region, a diameter of the nozzle portion 71, and a discharge pressure of the liquid chemical (corresponding to maximum combustion pressure in a predetermined volume of explosives used in the injector 1 of a low explosive type). The retention time is set, for example, between 3 to 20 seconds based on these data. This setting may be performed in advance by connecting a USB cable to the connector 4 to connect the injector 1 to a personal computer and transmitting setting information of the retention time from the personal computer to the controller 82. When the controller 82 receives the setting information of the retention time from the personal computer, the setting information is stored in CPU, RAM, and the like in the arithmetic processing unit 83. The controller 82 may separately include a storage unit such as a non-volatile memory that stores this setting information. Alternatively, a device that inputs these information (for example, an input device capable of selecting data by actuating button or setting by input) may be incorporated into the housing 2.

After the igniter 22 has been actuated to deliver the ejection solution into the target region, the controller 82 makes a predetermined notification regarding the first timing for canceling the contact state between the ejection port 77 and the surface of the target region to a user. In the present embodiment, the controller 82 determines whether the retention time has elapsed since the igniter 22 has actuated while the time when the ejection solution is delivered into the target region is regarded as the time when the igniter 22 is actuated (step S104). When the controller 82 determines that the retention time has elapsed (Yes in step S104), the controller 82 makes the predetermined notification (step S105) and terminates the processing. The predetermined notification is a notification regarding the first timing for canceling the contact state between the ejection port 77 and the surface of the target region. The controller 82 controls the speaker 81 to make the predetermined notification to the user. With this configuration, the injector 1 can maintain the contact state between the ejection port 77 and the surface of the target region for the retention time until the predetermined notification regarding the first timing is made to the user and therefore can suppress backflow of the ejection solution. A vibration unit including a vibration motor may be provided in the housing 2 of the injector 1, and the controller 82 may control the vibration unit to make the predetermined notification by vibration. The predetermined notification is made after the ejection solution has been delivered into the target region, and thus may be made by vibrating the housing 2 and the like.

### <Second embodiment>

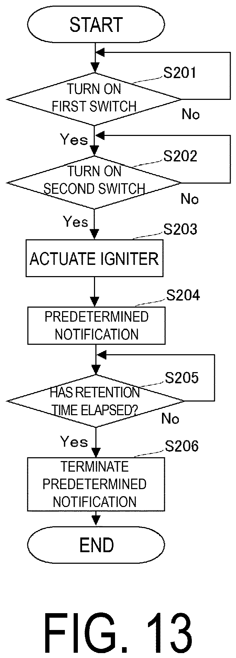

Next, a second embodiment of the injector 1 will be described with reference to FIG. 13. In the present embodiment, the controller 82 continuously generates a signal related to the predetermined notification during a time from the start of the imparting of the ejection energy by the plunger 80 after the piston 40 slides toward the distal end side to the first timing.

After the igniter 22 has been actuated to deliver the ejection solution into the target region, the controller 82 makes the predetermined notification regarding the first timing for canceling the contact state between the ejection port 77 and the surface of the target region to a user. FIG. 13 is a flowchart illustrating processing performed by the controller 82 during the operation of the injector 1. Since the respective processes in steps from S201 to S203 in the flowchart of FIG. 13 are the same as the respective processes in steps from S101 to S103 in the flowchart of FIG. 12, these descriptions will be omitted.

In step S204, the controller 82 makes the predetermined notification. Specifically, the controller 82 continuously generates the signal related to the predetermined notification at the same time as ignition operation, and transmits the signal to the speaker 81. With this configuration, the controller 82 controls the speaker 81 to make the predetermined notification. For example, the controller 82 controls the speaker 81 to output effect sounds such as buzzer, voices, and the like, and thus to make the predetermined notification. A lamp, a display device, or the like may be provided in the housing 2 of the injector 1 instead of the speaker 81. When the injector 1 includes a display device, the controller 82 may control the display device to make the predetermined notification by a countdown display indicating a remaining time of the retention time.

Next, the controller 82 determines whether the retention time has elapsed since the igniter 22 has actuated while the time when the ejection solution is delivered into the target region is regarded as the time when the igniter 22 is actuated (step S205). When the controller 82 determines that the retention time has elapsed (Yes in step S205), the controller 82 terminates the predetermined notification (step S206) and terminates the processing. The controller 82 stops transmitting the signal related to the predetermined notification to the speaker 81 and terminates the predetermined notification.

In the present embodiment, the controller 82 makes the predetermined notification during the retention time, and terminates the predetermined notification after the retention time has elapsed. With this configuration, the injector 1 can maintain the contact state between the ejection port 77 and the surface of the target region for the retention time until the predetermined notification regarding the first timing is terminated to the user and therefore can suppress backflow of the ejection solution.

### <Third embodiment>

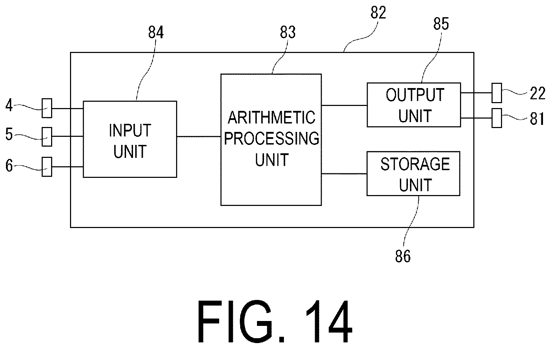

Next, a third embodiment of the injector 1 will be described with reference to FIG. 14. In the present embodiment, the injector 1 includes a storage unit 86 that stores first information pertaining to a target region and a user. As illustrated in FIG. 14, the storage unit 86 is included in the controller 82. However, the present invention is not limited to this configuration, and the storage unit 86 may be provided separately outside the controller 82.

The storage unit 86 is constituted of a non-volatile memory, and stores the first information. The first information includes a site of the target region, age and gender of a subject person, and the like. The controller 82 makes a predetermined notification based on the first information stored in the storage unit 86. For example, the first information is stored in the storage unit 86 in advance by connecting a USB cable to a connector 4 to connect the injector 1 to a personal computer and transmitting the first information from the personal computer to the controller 82. For example, the storage unit 86 has a data table for determining the retention time based on the first information, and the controller 82 references the data table based on the first information to determine the retention time and performs the processing illustrated in FIGS. 12 and 13 to make the predetermined notification. With this configuration, the injector 1 can determine the suitable retention time from the first information and make the predetermined notification, so that backflow of the ejection solution can be suppressed. In addition to using the personal computer to input the first information to the storage unit 86, such an input device that can select ages, administered sites, body data (height, weight, etc.) of the subject person, a specification of the driving part (igniter 22), etc. from a selection screen and set an optimal retention time may be incorporated into the housing 2, or the input device may be prepared as an external device, connected to the input unit 84 of the controller 82 when the first information is input, and input the first information to the storage unit 86.

### <Fourth embodiment>

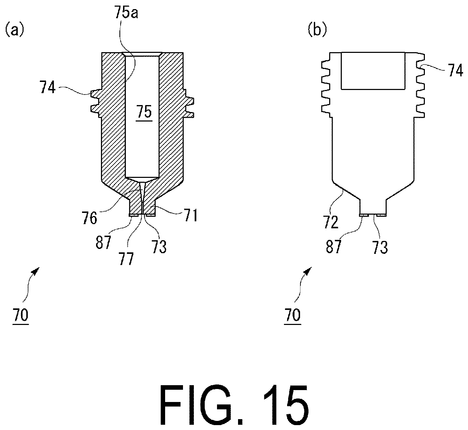

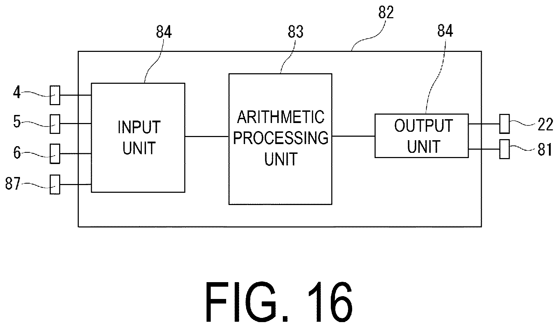

Next, a fourth embodiment of the injector 1 will be described with reference to FIGS. 15 and 16. FIG. 15 includes the diagram (a) on the left side that is a cross-sectional view of a container 70, and the diagram (b) on the right side that is an external view of the container 70. The injector 1 according to the present embodiment includes a sensor 87 disposed to surround an ejection port 77 on a distal end surface 73 of a nozzle portion 71. In the present embodiment, the sensor 87 acquires second information pertaining to a target region near the ejection port 77 in a contact state between the ejection port 77 and a surface of the target region. The second information is moisture, elasticity, thickness of the skin, and the like at a site of the target region.

The sensor 87 is removably attached to the container 70. The injector 1 includes an arm portion extending from the housing 2 to the ejection port 77 of the container 70 along a longitudinal direction of an injector assembly 10, and the sensor 87 may be attached to a distal end of the arm portion.

FIG. 16 is a block diagram of a controller 82 included in the injector 1 according to the present embodiment. The sensor 87 is connected to an input unit 84. The controller 82 makes a predetermined notification based on the second information acquired by the sensor 87. The controller 82 determines a retention time based on the second information, and performs processing illustrated in FIGS. 12 and 13 to make the predetermined notification. The injector 1 can determine the retention time from the second information and make the predetermined notification, so that backflow of an ejection solution can be suppressed. A storage unit 86 may also be provided in the controller 82 illustrated in FIG. 16. In this case, the controller 82 has a data table for determining the retention time based on the second information, and the controller 82 may reference the data table based on the second information to determine the retention time.

### <Modified example 1 of fourth embodiment>

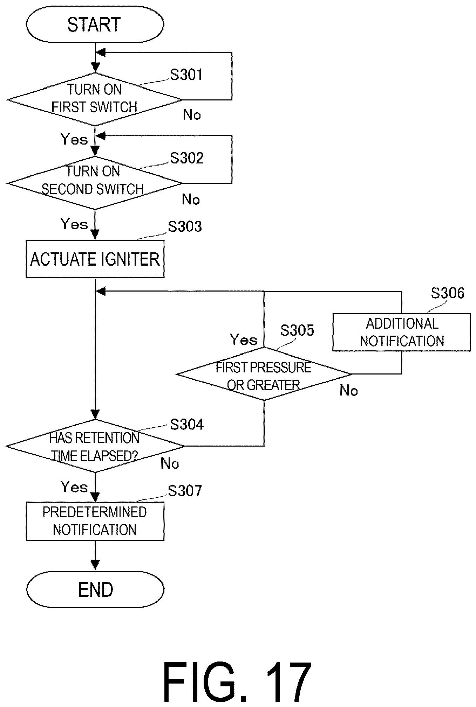

Next, a modified example 1 of the fourth embodiment of the injector 1 will be described with reference to FIGS. 15 to 17. In the present modified example, the sensor 87 is a pressure sensor capable of detecting the contact state between the ejection port 77 and the surface of the target region. In the controller 82, an igniter 22 is actuated, a piston 40 slides toward a distal end side to push a plunger 80 toward the distal end side, and when a detected value from the sensor 87 falls below a predetermined first pressure value until a retention time has elapsed (the first timing has elapsed) from the start of imparting of the ejection energy to the ejection solution, the controller 82 makes an additional notification to a user so that a pressing force of the ejection port 77 against the surface of the target region increases. The controller 82 makes the additional notification while controlling the speaker 81. This control will be described with reference to FIG. 17. FIG. 17 is a flowchart of processing performed by the controller 82. Since the respective processes in steps from S301 to S303 in the flowchart of FIG. 17 are the same as the respective processes in steps from S101 to S103 in the flowchart of FIG. 12, these descriptions will be omitted.

In step S304, the controller 82 determines whether the retention time has elapsed since the igniter 22 has been actuated. When the controller 82 determines that the retention time has not elapsed (No in step S304), the controller 82 determines whether the detected value from the sensor 87 is the predetermined first pressure value or greater. The predetermined first pressure value is the pressure required to deliver the ejection solution to the target region at the retention time or to prevent backflow of the delivered ejection solution. When the controller 82 determines that the detected value from the sensor 87 is not the predetermined first pressure value or greater (No in step S305), the controller 82 makes an additional notification (step S306). As described above, when the detected value from the sensor 87 falls below the predetermined first pressure value, the controller 82 makes an additional notification to the user to increase the pressing force of the ejection port 77 against the surface of the target region, and the additional notification is made in a manner distinguishable from a predetermined notification. The controller 82 controls the speaker 81 to output effect sounds such as buzzer and voices, and thus to make the additional notification. Instead of the speaker 81, a lamp, a display device, or the like may be provided in a housing 2 of the injector 1, and the controller 82 may control the lamp, the display device, or the like to make an additional notification. A combination of two or more of a speaker, a lamp, and a display device is provided in the housing 2, and the controller 82 may control these devices and make various notifications to the user.

On the other hand, when the controller 82 determines that the detected value from the sensor 87 is the predetermined first pressure value or greater (Yes in step S305), the controller 82 does not make an additional notification and repeats the determination in step S304 until the retention time has elapsed. Since the process in step S307 is the same as the process in step S105 in the flowchart of FIG. 12, the description will be omitted. The controller 82 may perform the processes in steps from S204 to S206 in the flowchart illustrated in FIG. 13 following the process in step S303.

The injector 1 can maintain the contact state between the ejection port 77 and the surface of the target region at the predetermined first pressure value or greater until the retention time has elapsed, so that backflow of the ejection solution can be suppressed.

### <Modified example 2 of fourth embodiment>

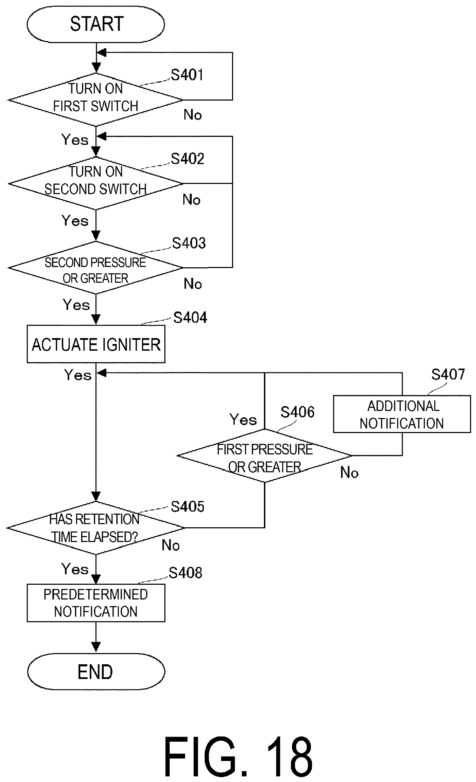

Next, a modified example 2 of the fourth embodiment of the injector 1 will be described with reference to FIGS. 15, 16, and 18. In the present modified example, the sensor 87 is a pressure sensor capable of detecting the contact state between the ejection port 77 and the surface of the target region, and the igniter 22 is allowed to operate when the detected value from the sensor 87 is a predetermined second pressure value or greater. The processing of the controller 82 in the present modified example will be described with reference to FIG. 18. FIG. 18 is a flowchart of processing performed by the controller 82. Since the respective processes in steps S401 and S402 in the flowchart of FIG. 18 are the same as the respective processes in steps S101 and S102 in the flowchart of FIG. 12, these descriptions will be omitted.

In step S403, the controller 82 determines whether the detected value from the sensor 87 has reached the predetermined second pressure value or greater. The predetermined second pressure value is the pressure required to deliver the ejection solution to the target region without leakage, and, for example, the pressure required to prevent formation of a gap between the target region and the ejection port 77 when a portion of the skin in the target region is ruptured with the kinetic energy of the ejection solution. When the controller 82 determines that the detected value from the sensor 87 is not the predetermined second pressure value or greater (No in step S403), the controller 82 does not transition to the process in step S404 of actuating the igniter, and returns to the process in step S402. At this time, the controller 82 may notify the user that the speaker 81 is controlled and the detected value from the sensor 87 does not reach the predetermined second pressure value and that the operation of the second switch 6 is required again. As described above, the injector 1 does not allow actuation of the igniter 22 if the detected value from the sensor 87 is less than the predetermined second pressure value. Since the respective processes in steps from S404 to S408 are the same as the respective processes in steps from S303 to S307 in the flowchart of FIG. 17, these descriptions will be omitted. The controller 82 may perform the processes in steps from S104 to S105 in the flowchart illustrated in FIG. 12 and the processes in steps from S204 to S206 in the flowchart illustrated in FIG. 13 following the process in step S403.

Since the injector 1 can actuate the igniter 22 and eject the ejection solution in the state in which the ejection port 77 and the surface of the target region are in contact with each other at the predetermined second pressure value required for delivering the ejection solution to the target region, the ejection solution can be delivered under the skin in the target region, so that backflow of the ejection solution can be suppressed.

### <Fifth embodiment>

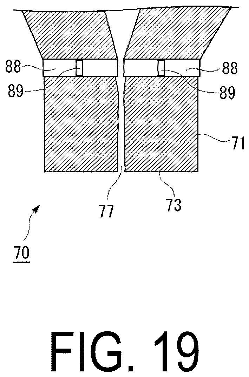

Next, a fourth embodiment of the injector 1 will be described with reference to FIG. 19. FIG. 18 is an enlarged cross-sectional view of a vicinity of a nozzle portion 71 of a container 70. The injector 1 according to the present embodiment includes a retreat portion 88 that retreats the nozzle portion 71 to the inside of the injector 1 to cancel a contact state between an ejection port 77 and a surface of a target region when a predetermined notification is made by a controller 82. The retreat portion 88 is disposed on a base end side of the nozzle portion 71, and is a space in which the nozzle portion 71 can be retreated to the base end side.

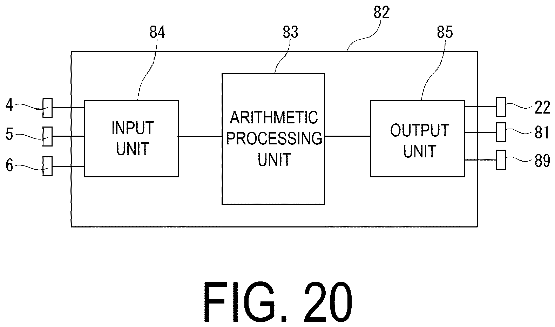

A movement mechanism 89 that moves the nozzle portion 71 toward a distal end side and the base end side is disposed in the retreat portion 88. A known actuator such as a microlinear actuator can be used for the movement mechanism 89. As illustrated in the block diagram of the controller 82 in FIG. 20, the movement mechanism 89 is connected to an output unit 85 and controlled by the controller 82. When the controller 82 makes the predetermined notification, the controller 82 controls the movement mechanism to allow the retreat portion 88 to retreat the nozzle portion 71 in order to cancel the contact state between the ejection port 77 and the surface of the target region. The injector 1 can cancel the contact state between the ejection port 77 and the surface of the target region, rather than by determination and operation by a user after the retention time has elapsed. The retreat itself of the nozzle portion 71 to the retreat portion 88 may be the predetermined notification. In addition, a rim surrounding the nozzle portion 71 around the container 70 is provided, so that the ejection port 77 slightly protrudes from a distal end of the rim before ejection of an ejection solution or the rim and the ejection port 77 are flush. After the retention time has elapsed, the nozzle portion 71 may be stored in the rim, only a distal end portion of the rim may be in contact with the target region, and the ejection port 77 may be separated from the target region.

According to the injector 1 according to the embodiment described above, a user can be notified of the retention time after ejection of the ejection solution or a timing of separation of the ejection port 77 from the surface of the target region, and therefore, it is possible to suppress backflow of the ejection solution and improve reliability of delivery of the ejection solution.

Each aspect disclosed in the present specification can be combined with any other feature disclosed herein.

### [Reference Signs List]

- 1: Injector
- 2: Housing
- 2a: Grip portion
- 3: Power cable
- 4: Connector
- 5: First switch
- 6: Second switch
- 10: Injector assembly
- 20: Actuator
- 21: Body
- 22: Igniter
- 30: Attachment
- 31: Body
- 40: Piston
- 50: Plunger rod
- 51: Shaft member
- 52: Diameter reduced portion
- 60: Stopper portion
- 70: Container
- 71: Nozzle portion
- 75: Accommodating space
- 76: Flow path
- 77: Ejection port
- 80: Plunger
- 81: Speaker
- 82: Controller
- 83: Arithmetic processing unit
- 84: Input unit
- 85: Output unit
- 86: Storage unit
- 87: Sensor
- 88: Retreat portion
- 89: Movement mechanism

## Claims

1. A needleless injector that ejects an intended injection substance from an ejection port into a target region in a state in which the ejection port is in contact with a surface of the target region, the needleless injector comprising:
a housing part that has an accommodating space that accommodates the intended injection substance;
a nozzle portion that defines a flow path for guiding the intended injection substance accommodated in the housing part to the ejection port;
a driving part that imparts ejection energy for ejecting the intended injection substance;
a pressurizing portion that pressurizes the intended injection substance accommodated in the accommodating space via a propellant disposed to move or deform in a predetermined direction inside the needleless injector by imparting the ejection energy; and
a notification unit that, after the ejection energy is imparted by the driving part and the intended injection substance is delivered into the target region, makes a predetermined notification regarding a first timing for canceling a contact state between the ejection port and the surface of the target region to a user.

2. The needleless injector according to claim 1, wherein the notification unit continuously generates a signal related to the predetermined notification during a time from a start of the imparting of the ejection energy by the driving part to the first timing.

3. The needleless injector according to claim 1 or 2, further comprising a storage unit that stores first information pertaining to the target region and the user,
wherein the notification unit makes the predetermined notification based on the first information stored in the storage unit.

4. The needleless injector according to claim 1 or 2, further comprising a predetermined sensor that acquires second information pertaining to the target region near the ejection port in the contact state between the ejection port and the surface of the target region,
wherein the notification unit makes the predetermined notification based on the second information acquired by the predetermined sensor.

5. The needleless injector according to any one of claims 1 to 4, further comprising a pressure sensor that detects the contact state between the ejection port and the surface of the target region,
wherein the notification unit makes an additional notification to a user so that a pressing force of the ejection port against the surface of the target region is increased when a detected value from the pressure sensor falls below a predetermined first pressure value until the first timing has elapsed from the start of imparting of the ejection energy by the driving part.

6. The needleless injector according to claim 5, wherein the driving part is allowed to operate when the detected value from the pressure sensor is a predetermined second pressure value or greater.

7. The needleless injector according to any one of claims 1 to 6, further comprising a retreat portion that retreats the nozzle portion to the inside of the needleless injector to cancel the contact state between the ejection port and the surface of the target region when the notification unit makes the predetermined notification.
